(19) Europäisches Patentamt
European Patent Office
Office européen des brevets

(11) Numéro de publication : **0 379 423 B1**

(12) # FASCICULE DE BREVET EUROPEEN

(45) Date de publication du fascicule du brevet : **23.06.93 Bulletin 93/25**

(51) Int. Cl.$^5$ : **C07C 227/00,** C07C 229/42

(21) Numéro de dépôt : **90400120.3**

(22) Date de dépôt : **16.01.90**

(54) **Nouveau procédé d'obtention d'un acide phénylpropionique.**

(30) Priorité : **16.01.89 FR 8900522**

(43) Date de publication de la demande : **25.07.90 Bulletin 90/30**

(45) Mention de la délivrance du brevet : **23.06.93 Bulletin 93/25**

(84) Etats contractants désignés : **AT BE CH DE DK ES FR GB GR IT LI LU NL SE**

(56) Documents cités :
**CH-A- 525 882**
**DE-A- 1 913 742**
**DE-A- 2 223 855**
**FR-A- 2 193 579**

(56) Documents cités :
**CHIMICA THERAPEUTICA, vol. 14, no. 3, mai-juin 1979, pages 207-214; B. DUMAITRE et al: "Synthèse de quelques dérivés des acides amino-4 phénylacétique et (amino-4 phényl)-2 propionique possédant des activités analgésiques et anti-inflammatoires"**

(73) Titulaire : **BOUCHARA S.A.**
**68, Rue Marjolin**
**F-92300 Levallois-Perret (FR)**

(72) Inventeur : **Perrin, Claude**
**05, Rue de l'avenir**
**F-91400 Orsay (FR)**

(74) Mandataire : **Burtin, Jean-François Bouchara S.A.**
**68, rue Marjolin B.P. 67**
**F-92302 Levallois Perret (FR)**

EP 0 379 423 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte à l'obtention d'un acide 2-phényl propionique par un nouveau procédé.

Elle a plus particulièrement pour objet l'obtention d'acide 2- phényl propioniques dont le noyau phényle est substitué par un radical aminé.

Elle a spécifiquement pour objet un nouveau procédé de synthèse des acides 2(R-amino phényl) propionique et, en particulier, de l'acide 2-(4-méthallylamino-phényl) propionique. En effet, la littérature, comme par exemple le brevet US 3,957,850 a montré la difficulté d'effectuer l'alcoylation de la fonction amine portée par de tels acides phénylpropionique. Or, il s'est avéré que la mono-alcoylation sélective de ces acides 2-(4-amino-phényl) propionique est importante dans la mesure où elle amène une augmentation notable de l'activité pharmacologique et/ou une réduction du degré de toxicité.

Au contraire, les dérivés NN-disubstitués sont, en général, significativement moins actifs et peuvent, de ce fait, constituer une charge supplémentaire dont l'élimination est souvent malaisée et amène des pertes sensibles de rendement aux stades ultimes de la synthèse.

C'est pourquoi, il s'est révélé important de trouver une nouvelle méthode de synthèse qui n'amène qu'une formation limitée de dérivés dialcoylés à l'azote ou qui en permette la séparation dans des conditions beaucoup plus aisées.

Ce problème a pu être résolu selon l'invention par un procédé qui consiste en ce que l'on soumet un dérivé 2(4- aminophényl) propionique de formule II

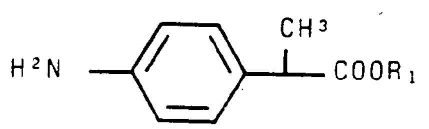

(II)

dans laquelle $R_1$ est un radical alcoyle ayant de 1 à 6 atomes de Carbone ou de l'hydrogène
en solution dans un solvant organique inerte non miscible à l'eau et non polaire, à l'action d'un agent d'alcoylation en phase hétérogène, en présence d'un catalyseur de transfert de phase et d'un agent basique en milieu aqueux pour obtenir un acide 2-(4-R aminophényl) propionique de formule générale I

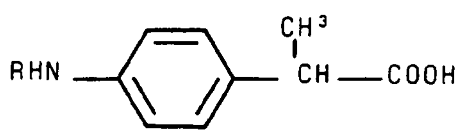

(I)

dans laquelle R est un radical alcoyle inférieur, éventuellement substitué par un hydroxyle, un radical alcényle inférieur ayant de 2 à 6 atomes de Carbone, un radical alcynyle inférieur ayant de 2 à 4 atomes de Carbone, un radical aralcoyle inférieur.

D'une manière préférée, le radical R est un méthallyle. Il peut également être un radical alcényle comme allyle, un radical aralcoyle comme benzyle, un radical alcoyle substitué comme β-hydroxyéthyle.

Le catalyseur de transfert de phase est un sel d'ammonium quaternaire ou de phosphonium,soluble dans les deux types de solvants. On utilise, de préférence, le chlorure de méthyltrioctyl ammonium commercialisé sous la dénomination ALIQUAT 336 ainsi que d'autres sels d'ammonium ou de phosphonium, tels que le bromure de méthyl triphényl phosphonium, le chlorure de triméthyl phényl ammonium, le bromure de tétrabutyl ammonium et le chlorure de benzyl tributyl ammonium.

La quantité de catalyseur utilisée est d'environ 10 g par mole d'acide 2-(4-amino phényl)propionique ou d'ester d'alcoyle de celui-ci, mis en oeuvre. La concentration en acide 2-(4-amino phényl) propionique ou en ester de cet acide est d'environ une mole pour 400 ml de solvant organique non miscible à l'eau.

La concentration en agent d'alcoylation qui est de préférence un halogénure de méthallyle, comme le chlorure de méthallyle ou le bromure de méthallyle, est de 1 à 1,4 mole par mole d'acide 2-(4-amino phényl) propionique ou d'ester d'alcoyle de cet acide. De préférence, on utilise de 1,1 à 1,4 mole d'agent de méthallylation par mole d'acide 2-(4-aminophényl)propionique ou d'un de ses esters d'alcoyle.

L'acide 2-(-4 méthallylamino phényl) propionique formé dans cette réaction reste dans la phase organique car la quantité d'agent basique est pratiquement celle de la stoechiométrie et ceci explique que le produit récupéré soit directement l'acide 2-(4- méthallyl amino phényl) propionique lui-même et non un de ses sels. Dans ces conditions, il n'y a pas de réaction de salification et l'isolement de l'acide est facilité.

L'agent basique susceptible de fixer l'hydracide ou l'anion formé au cours de la réaction d'alcoylation est une base forte, de préférence un hydroxyde de métal alcalin comme la soude. La concentration utilisée est de préférence de 1 à 1,1 de mole de base pour 1 mole d'acide ou d'ester de l'acide 2-(-4 amino phényl) propionique, en solution dans l'eau à une concentration de l'ordre de 10 %.

La réaction s'effectue à une température inférieure à celle de l'ébullition du solvant organique et, de préférence, entre 80 et 85°. Il est préférable d'opérer sous atmosphère inerte, comme par exemple par barbottage d'azote.

La durée de la réaction est au minimum de deux heures. La poursuite de la réaction au-delà de 4 heures n'apporte pas d'avantage ou un meilleur rendement.

Après réaction, on sépare la phase organique surnageante et on la lave à l'eau. La phase organique est extraite par de la soude environ 1 N. Elle est ensuite acidifiée à pH 1 au moyen d'acide chlorhydrique 1 N. Les produits secondaires (en particulier des dérivés dialcoylés) y sont insolubles et éliminés par extraction avec un solvant organique.

Cette solution acide est traitée au noir activé, filtrée et amenée à pH 3 par addition de soude ou d'un sel de métal alcalin d'un acide faible.

L'acide 2-(-4 alcoylaminophényl) propionique qui cristallise est séparé par filtration. Il est lavé à l'eau et séché.

On a constaté avec intérêt que lorsque l'on part d'un ester d'alcoyle de l'acide 2-(4- aminophényl) propionique, la réaction d'alcoylation menée dans ces conditions s'accompagne de la saponification de la fonction ester de telle sorte que le produit obtenu est directement l'acide 2- (4-alcoyl aminophényl) propionique.

L'acide 2-(4- méthallylamino phényl) propionique ainsi obtenu est un produit connu (B. DUMAITRE cf. Chimie Thérapeutique 14 (1979) p. 207-214) dont les propriétés analgésiques et anti-inflammatoires ont fait l'objet de nombreuses publications.

La production de l'acide 2-(4- méthallylaminophényl) propionique avait cependant été jusqu'ici freinée car les méthodes de synthèse butaient toutes sur un stade difficile à réaliser : l'alcoylation du dérivé aminé par un halogénure de méthallyle. Ce stade consiste a à soumettre précocement ou non,le dérivé amino à l'action d'un agent de méthallylation et il s'était jusqu'ici avéré impossible d'éviter la formation simultanée d'un dérivé mono méthallylamino et d'un dérivé diméthallyl amino. On a constaté, en particulier, qu'un bloquage partiel labile de la fonction amine qui aurait pû éviter cette réaction secondaire, était rapidement inopérant car difficile à éliminer ultérieurement. De ce fait, les différentes méthodes de synthèse conduisaient inéluctablement à la formation de ce sous-produit, sans intérêt thérapeutique, et dont l'élimination nécessitait une étape supplémentaire et une baisse sensible du rendement global de la synthèse.

Les exemples suivants illustrent l'invention.

Exemple 1

Dans un réacteur muni d'une bonne agitation, d'un réfrigérant, d'un thermomètre et d'une arrivée d'azote, placer :

- 165 g (1 mole) d'acide 2-(4-aminophényl) propionique
- 400 $cm^3$ de Toluène
- 100 g de chlorure de méthallyle (1,1 mole)
- 10 g d'Aliquat 336
- 250 $cm^3$ d'eau
- 92,5 $cm^3$ de lessive de soude à 32 % (1 mole)

Chauffer 3 heures à 85°.

Décanter, laver deux fois la solution toluénique avec de l'eau. Extraire cette phase toluénique avec 1 l de soude 1 N. Verser la solution alcaline sur 1,5 litres d'acide chlorhydrique 1 N-(pH = 0,8). Extraire trois fois avec du Toluène Ajouter du charbon (filtrer). Ramener le pH à 3 par addition lente de soude 1 N sous très bonne agitation. Essorer le précipité formé, le laver à l'eau et sécher.

On isole ainsi 140 g (rendement = 64 %) de cristaux blanc crème. F⏝ = 107°.

Exemple 2

D'une manière identique à l'exemple 1, on obtient 135 g d'acide 2-(4-méthallyl-amino phényl) propionique à partir de 179 g de 2-(4-amino phényl) propionate de méthyle en utilisant de l'Aliquat 336.

Exemples 3 à 7

D'une manière identique à l'exemple 1, on obtient l'acide 2-(4-méthallyl aminophényl) propionique à partir de l'acide 2-(4-amino phényl) propionique en utilisant les catalyseurs de transfert de phase suivants :

| Exemple | Catalyseur utilisé | Rendement |
|---|---|---|
| 3 | T E B A | 57 % |
| 4 | Méthyl triphényl phosphonium bromure | 58 % |
| 5 | Phényl triméthyl ammonium chlorure | 57,5 % |
| 6 | Tétrabutyl ammonium bromure | 58,5 % |
| 7 | Benzyl tributyl ammonium chlorure | 62 % |

## Revendications

1. Un procédé d'obtention d'acides 2-phényl propionique substitués par un radical aminé caractérisé en ce que l'on soumet un dérivé 2-(4- aminophényl) propionique de formule II

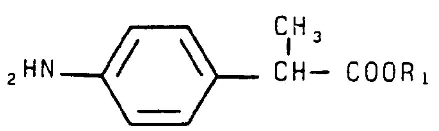 (II)

dans laquelle $R_1$ est un radical alcoyle ayant de 1 à 6 atomes de Carbone ou de l'hydrogène.
en solution dans un solvant organique inerte, non miscible l'eau et non polaire, à l'action d'un agent d'alcoylation en phase hétérogène en présence d'un catalyseur de transfert de phase et d'un agent basique en milieu aqueux pour obtenir un acide 2-(4.R. aminophényl) propionique de formule générale I

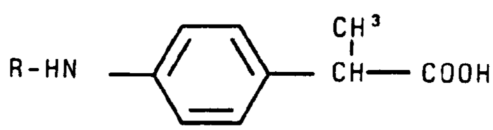 (I)

dans laquelle R est un radical alcoyle inférieur éventuellement substitué par un hydroxyle, un radical alcényle inférieur, un radical alcynyle inférieur ou un radical aralcoyle inférieur.

2. Un procédé selon la revendication 1° dans lequel le radical R est le radical méthallyle.

3. Un procédé selon la revendication 1° dans lequel le solvant organique, non miscible à l'eau, est un carbure aromatique liquide.

4. Un procédé selon la revendication 1° dans lequel le catalyseur de transfert de phase est un sel d'ammonium quaternaire soluble à la fois dans les solvants hydrophobes et dans l'eau.

5. Un procédé selon la revendication 1° dans lequel le catalyseur de transfert de phase est un sel de triphénylalcoyl phosphonium.

6. Un procédé selon la revendication 4° dans lequel le catalyseur de transfert de phase est le bromure de méthyltrioctyl ammonium.

7. Un procédé selon l'une des revendications 1 à 6 dans lequel la quantité de catalyseur de transfert de phase introduite dans le milieu réactionnel est de l'ordre de 10 g par mole d'acide 2-(4- aminophényl) propionique.

8. Un procédé selon l'une des revendications 1° à 7° dans laquell la concentration en dérivé 2-(4- aminophényl) propionique est d'environ 1 mole pour 400 ml de solvant organique non miscible à l'eau,

9. Un procédé selon l'une des revendications 1° à 8° dans lequel la concentration en agent d'alcoylation est comprise entre 1 et 1,4 mole par mole d'acide 2-(4-aminophényl) propionique.

10. Un procédé selon la revendication 9° dans lequel la concentration en agent de méthallylation varie de 1,1 à 1,4 mole par mole d'acide 2-(4- aminophényl) propionique.

**Patentansprüche**

1. Verfahren Zur Herstellung von mit einer Aminogruppe substituierten 2-phenyl propionsäure dadurch gekennzeichnet daß ein Derivat der 2-(4-Aminophenyl) propionsäure der allgemeinen Formel II

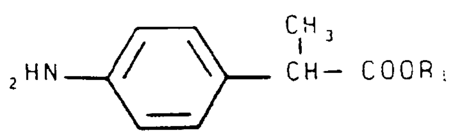

(II)

worin $R_1$ ein Alkyl rest mit 1 bis 6 Kohlenstoff atomen oder Wasserstoff ist
als Lösung in einem Wasser-unmischlaren und nicht-polaren, inerten, organischen Lösungsmittel
die Einwirkung eines Alkylierungsmittel in der Form einer heterogenen Phase, in Gegenwart eines Phase transfert-Katalyst und eines basischen Mittel in wassrigen Medium unterwirft, um eine 2-( 4-R Aminophenyl ) propionSäure der allgemeinen Formel I

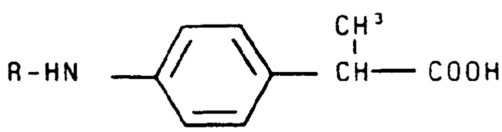

(I)

worin R einen gegebenenfalls mit einem Hydroxy substituierten niedrig Alkyl Rest, ein niedrig Alkenyl Rest, ein niedrig Alkylnyl Rest oder einen Aryl(niedrig Alkyl) Rest bedeutet
zu gewinnen

2. Verfahren nach Anspruch 1°, worin das Rest R einen Methallylrest ist

3. Verfahren nach Anspruch 1°, worin das organisches, mit wasser unmischbares Lösungsmittel, eines flüssiges aromatisches Kohlenwasserstoff ist

4. Verfahren nach Anspruch 1°, worin das Phase-Transfert-Katalyst eine quaternäre Ammoniumsalz ist, der gleichzeitig, in der hydrophoben Lösungsmitteln und ins Wasser löslisch ist

5. Verfahren nach Anspruch 1°, worin das Phase-Transfert-katalyst eine Salz von Triphenylalkyl phosphonium ist

6. Verfahren nach Anspruch 4°, worin das Phase-Transfert-katalyst das Methyl(Trioctyl)Ammonium Bromid ist

7. Verfahren nach Anspruch 1 bis 6° worin die in das Reaktionsmittel eingebrachte Menge an Phase-Transfert-katalyst der Ordnung von 10 g per mol 2-(4-Aminophenyl) propionsäure beträgt

8. Verfahren nach eine der Ansprüch 1 bis 7° worin die Konzentration an 2-(4-Aminophenyl) propionsäure ungefahr 1 Mol für 400 ml von wasser-unmischbaren organischen Lösungsmittel beträgt

9. Verfahren nach eine der Ansprüch 1 bis 8° worin die Konzentration an alkylierendes Mittel zwischen 1 und 1,4 Mol pro Mol 2-(4-Aminophenyl) propionsäure liegt

10. Verfahren nach Ansprüch 9° worin die Konzentration an methallylierendes Mittel von 1,1 bis 1,4 Mol pro Mol von 2-(4-Aminophenyl) Propionsäure variiert.

## Claims

1. A process for producing 2-phenyl propionic acids which are substituted with an amino group, wherein a 2-(4-aminophenyl) propionic acid of formula II

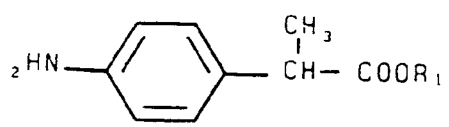

(II)

wherein $R_1$ is an alkyl radical having from 1 to 6 carbon atoms or a hydrogen dissolved in an inert organic water-immiscible and non-polar solvent is submitted to the action of an alkylating agent in heterogeneous phase, in the presence of a phase transfert catalyst and a basic agent in an aqueous medium, to produce a 2-(4-R aminophenyl) propionic acid of general formula I

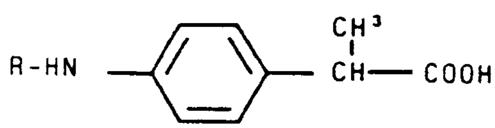

(I)

wherein R is a lower alkyl group which may optionally be substituted with a hydroxy, a lower alkenyl group, a lower alkynyl group or an aryl lower alkyl group.

2. A process according to claim 1° in which the group R is a methallyl group

3. A process according to claim 1° in which the organic, water-immiscible solvent is a liquid aromatic hydrocarbon

4. A process according to claim 1° in which the phase transfert catalyst is a quaternary ammonium salt which is soluble at the same time, in the hydrophobic solvents and in water

5. A process according to claim 1° in which the phase transfert catalyst is a triphenylalkyl phosphonium salt

6. A process according to claim 4° in which the phase transfert catalyst is methyl trioctyl ammonium bromide

7. A process according to anyone of claims 1 to 6° in which the amount of phase transfert catalyst introduced in the reaction medium, is of the order of 10 g per mol of 2-(4-aminophenyl) propionic acid

8. A process according to anyone of claims 1 to 7°, in which the content into 2-(4-aminophenyl) propionic acid is about 1 mol for 400 ml of water-immiscible organic solvent

9. A process according to anyone of claims 1 to 8° in which the content of alkylating agent ranges between 1 and 1,4 mol per mol of 2-(4-aminophenyl) propionic acid

10. A process according to claim 9° wherein the content of methallylating agent ranges from 1,1 to 1,4 mol per mol of 2-(4-aminophenyl) propionic acid